# EUROPÄISCHE PATENTSCHRIFT

(11) **EP 1 067 862 B1**
(45) Veröffentlichungstag und Bekanntmachung des Hinweises auf die Patenterteilung: **26.05.2004**
(21) Anmeldenummer: 99917876.7
(22) Anmeldetag: 26.03.1999
(51) Int. Cl.: A61B 5/00, G06F 19/00

(54) **VERFAHREN ZUR KONTROLLE DER INSULINMEDIKATION**
METHOD FOR REGULATING INSULIN MEDICATION
PROCEDE POUR CONTROLER LE TRAITEMENT PAR INSULINE

(30) Priorität: 31.03.1998 DE 19814219
(43) Veröffentlichungstag der Anmeldung: 17.01.2001
(73) Patentinhaber: Roche Diagnostics GmbH, 68298 Mannheim (DE)
(72) Erfinder: WERNER, Karl, D-69168 Wiesloch (DE); BLASBERG, Peter, D-69469 Weinheim (DE); MÜLLER, Wilfried, D-66871 Konken (DE)
(74) Vertreter: Weber, Manfred, Dr.
(86) Internationale Anmeldenummer: PCT/EP1999/002074
(87) Internationale Veröffentlichungsnummer: WO 1999/049777

(56) Entgegenhaltungen:
- EP-A- 0 290 683
- EP-A- 0 462 466
- EP-A- 0 483 595
- DE-A- 2 758 467
- GB-A- 2 218 831

## Beschreibung

Die vorliegende Erfindung befaßt sich mit der Aufgabe, dem Diabetiker oder einer Betreuungsperson eine Möglichkeit an die Hand zu geben, eine Medikation mit Insulin auf die spezifischen Bedürfnisse des Diabetikers anzupassen.

Im Stand der Technik sind Blutglukosemeßgeräte bekannt, die eine sogenannte Tagebuchfunktion besitzen. Ein derartiges Gerät ist beispielsweise Accutrend DM der Firma Boehringer Mannheim. Mit diesem Gerät kann der Diabetiker seine Blutglukosekonzentrationen bestimmen und abspeichern. Durch den zeitlichen Verlauf der gemessenen Blutglukosewerte kann der Patient eine Vorstellung davon gewinnen, ob seine Therapierung mit Insulin geeignet ist. Ziel der Therapierung ist es, die Blutglukose in einem Normbereich zu halten, der etwa zwischen 80 und 180 mg/dl liegt. Sinkt der Blutzuckerspiegel unterhalb eines Wertes von 50 mg/dl so wird dieses als Hypoglykämie bezeichnet, die für den Patienten eine Gefahr darstellt, da er in seinen kognitiven Leistungen behindert ist oder sogar in den sogenannten hypoglykämischen Schock fällt, der zum Tode führen kann. Blutzuckerwerte oberhalb von 250 mg/dl hingegen sind unerwünscht, da sie zu Spätschäden, wie z.B. Diabetikerfüßen oder Erblindungen, führen. Neue Therapieformen erlauben dem Patienten bedarfsgerecht Mahlzeiten, Insulin und körperliche Aktivitäten zu dosieren. Zur erfolgreichen Durchführung müssen vom Diabetiker jedoch Strategien zur Insulintherapie erlernt werden. Mit dem im Stand der Technik bekannten System ist es für den Patienten bereits in einem gewissen Maße möglich mitzuverfolgen, welchen Einfluß Nahrungsaufnahme, körperliche Aktivitäten und Insulinverabreichungen auf seinen Blutzuckerspiegel nehmen. Die Systeme des Standes der Technik geben dem Benutzer jedoch keine einfache Möglichkeit, die vorgenommene Therapie durch Insulinverabreichung systematisch zu kontrollieren und Maßnahmen zur Verbesserung der Therapie zu ergreifen. Erschwerend kommt hinzu, daß sich die Insulinsensitivität des Patienten während des Tages ändert.

Dokument GB-A-2 218 831 beschreibt eine Vorrichtung zur Kontrolle der Insulinmedikation mit einer Eingabeeinheit zur Eingabe von Blutzuckerwerten, Broteinheiten und Insulineinheiten.

Aufgabe der vorliegenden Erfindung war es daher, dem Diabetiker oder einer Betreuungsperson eine Möglichkeit an die Hand zu geben, die Therapierung eines Diabetikers einfacher zu kontrollieren und zu systematisieren. Die vorliegende Erfindung schlägt ein Verfahren vor, bei dem Blutzuckerwerte, die jeweils vor und nach Mahlzeiten bestimmt wurden, ins Verhältnis zu einem Quotienten aus Insulingaben und Broteinheiten gesetzt werden.

Die vorliegende Erfindung betrifft ein Verfahren zur Kontrolle der Insulinmedikation mit den Schritten
a) Zurverfügungstellen von Datensätzen mit den folgenden Daten:
   ein Blutzuckerwert (BVᵢ), der vor einer Mahlzeit ermittelt wurde,
   ein Blutzuckerwert (BNᵢ), der nach der Mahlzeit ermittelt wurde,
   die Broteinheiten (BEᵢ), die bei der Mahlzeit aufgenommen wurden,
   die Insulineinheiten (Iᵢ), die zwischen Ermittlung der Blutzuckerwerte BVᵢ und BNᵢ verabreicht wurden,
b) Bildung von Blutzuckerdifferenzen ΔBᵢ aus den Blutzuckerwerten vor und nach der Mahlzeit für die einzelnen Datensätze,
c) Bildung eines Quotienten Qᵢ aus Insulineinheiten (Iᵢ) und Broteinheiten BEᵢ,
d) Auftragen von Punkten Pᵢ (ΔBᵢ; Qᵢ) in einem Koordinatensystem mit einer ersten Koordinate, für die Blutzuckerdifferenzen (ΔBᵢ)und einer zweiten Koordinate, für die Quotienten (Qᵢ),
e) Ausgabe des Koordinatensystems mit den Punkten auf einem Ausgabegerät.

Die vorliegende Erfindung ermöglicht dem Benutzer eine Visualisierung des Einflusses von Mahlzeiten und Insulingaben auf seinen Blutzuckerspiegel. Hierzu werden Daten, die die Broteinheiten von Mahlzeiten betreffen sowie die Insulineinheiten, die verabreicht werden, verwendet. Dies sind die Hauptfaktoren, die einen Einfluß auf den Blutzuckerspiegel nehmen. Neben diesen Einflußgrößen spielen noch körperliche Aktivitäten sowie physiologische Faktoren eine Rolle für die Veränderung der Blutglukosekonzentration. Sportliche Aktivitäten verbrauchen Blutzucker, so daß dieser Effekt durch Subtraktion entsprechende Äquivalente von den bei der Mahlzeit aufgenommenen Broteinheiten erfolgen kann.

Die im Rahmen dieser Erfindung verwendeten Broteinheiten (BE) sind im Stand der Technik gebräuchlich. Es existieren beispielsweise Tabellen, die angeben, wieviele Broteinheiten für eine definierte Menge eines bestimmten Lebensmittel veranschlagt werden müssen. Der Diabetiker kann mit Hilfe derartiger Tabellen errechnen, wieviele Broteinheiten eine bestimmte Mahlzeit umfaßt. Der verwendete Begriff Broteinheiten soll auch andere Mengenangaben umfassen, die charakteristisch für die aus der Nahrung gebildete Menge an Kohlenhydraten ist und die durch das verabreichte Insulin abgebaut werden soll. Eine Berechnung von Broteinheiten aus der Menge aufgenommener Kohlenhydrate, sowie dem Einfluß von Ballaststoffen, Fetten und Eiweißen ist in dem Buch "Diabetologie für praktische Ärzte und Kliniker" von B. Knick und J. Knick. In der 3. überarbeiteten und erweiterten Auflage findet sich die entsprechende Passage in Kapitel 4.2.

Die im Rahmen der Erfindung genannten Insulineinheiten (I) sind ebenfalls im Stand der Technik hinlänglich bekannt. Eine Definition der Internationalen Einheit für Insulin findet sich beispielsweise in Kapitel 5.4 des o.g. Buches "Diabetologie ...". Im Rahmen der vorliegenden Erfindung wird der Begriff "Insulineinheiten" jedoch nicht beschränkt auf Internationale Einheiten verwendet, sondern wird stellvertretend für Einheiten verwendet, die die Spezifizierung einer Insulinmenge ermöglichen. Der Diabetiker verwendet heutzutage annähernd ausschließlich standardisiertes Insulin, von dem bekannt ist, welche Menge injiziert werden muß, um eine gewünschte Zahl von Insulineinheiten zu verabreichen. In der Praxis wird dem Diabetiker eine derartige Berechnung sogar erspart, da die Spritzensysteme zur Verabreichung von Insulin auf eine gewünschte Insulinmenge eingestellt werden können, die dann exakt und sicher verabreicht wird. In der Praxis existieren verschiedene Arten von Insulin, die sich in ihrem Wirkprofil unterscheiden, in der das Insulin dem Körper zur Verfügung gestellt wird. Im Rahmen der Erfindung ist es vorgesehen, daß der Datensatz ebenfalls eine Information über die Art des Insulins trägt, so daß der Benutzer auch herausfinden kann, welches Insulin bzw. welche Kombination der verfügbaren Insulintypen für ihn am besten geeignet sind. Hierzu können die im Koordinatensystem aufgetragenen Punkte beispielsweise entsprechend der Art des Insulins gekennzeichnet werden.

Aus dem aufgenommenen Broteinheiten und den Insulineinheiten, die zur Kompensation der Broteinheiten verabreicht wurden, wird ein Quotient gebildet. In der Regel wird der Quotient als I/BE gebildet, es ist erfindungsgemäß jedoch auch das reziproke Verhältnis möglich. Vorzugsweise wird der ermittelte Quotient auf seine Plausibilität überprüft, bevor er im Rahmen des erfindungsgemäßen Verfahrens weiterverarbeitet wird. Hierzu wird beispielsweise überprüft, ob der Quotient positiv ist und ob er in einem bestimmten Bereich (z.B. von 0.1 bis 10) liegt.

Für die im Rahmen der Erfindung verwendeten Datensätze sind neben aufgenommenen Broteinheiten und der verabreichten Insulinmenge auch gemessene Blutzuckerkonzentrationen bzw. Blutzuckerwerte notwendig, die vor und nach der jeweiligen Mahlzeit ermittelt wurden. Der vor der Mahlzeit ermittelte Blutzuckerwert sollte kurz vor der Mahlzeit vorzugsweise weniger als 1 Stunde vor Beginn der Mahlzeit gemessen worden sein. Vorzugsweise werden zur weiteren Auswertung nur solche Messungen zugelassen, bei denen sich der Blutzuckerwert vor der Mahlzeit im Normbereich (80 bis 180 mg/dl) befindet.

Für den Blutzuckerwert nach der Mahlzeit gilt, daß dieser spätestens vor der nächsten Mahlzeit gemessen werden sollte. Die Bestimmung derartiger Blutzuckerwerte ist im Stand der Technik hinlänglich bekannt, so daß an dieser Stelle nicht näher darauf eingegangen wird. Es sei lediglich darauf hingewiesen, daß neben der weithin üblichen Bestimmung mit Kapillarblut, das aus einer Fingerbeere gewonnen wird, auch andere invasive und nicht-invasive Verfahren zur Bestimmung der Blutzuckerwerte möglich sind.

Neben den beschriebenen Insulingaben zur Kompensation der bei Mahlzeiten aufgenommenen Broteinheiten kann vom Diabetiker auch Insulin zur Kompensation zu hoher Blutzuckerwerte injiziert werden (sogenanntes Korrekturinsulin). Diese Injektionen können prinzipiell während des gesamten Tages erfolgen. Im Rahmen der vorliegenden Erfindung wird dieses Korrekturinsulin nicht berücksichtigt, wenn es ausreichend lange vor der Mahlzeit und der entsprechenden Blutzuckermessung vor der Mahlzeit verabreicht wurde. In diesem Fall kann davon ausgegangen werden, daß das Korrekturinsulin bereits im wesentlichen wirksam geworden ist und seine Auswirkung auf den Blutzuckerwert nach der Messung gering ist. Wird das Korrekturinsulin jedoch zusammen oder in kurzem zeitlichen Abstand zu der Injektion vor der Mahlzeit verabreicht, so soll es im Rahmen der Erfindung bei der Berechnung der verabreichten Insulindosis mit berücksichtigt werden. Es werden jedoch, wie bereits vorhergehend beschrieben, vorzugsweise nur solche Datensätze zur Auswertung bzw. Auftragung verwendet, bei denen der Blutzuckerwert vor der Mahlzeit im Normbereich liegt.

Aus den Blutzuckerwerten vor (BVᵢ) und nach (BNᵢ) der Mahlzeit werden Differenzen ΔBᵢ gebildet. Vorzugsweise ist ΔBᵢ gleich BNᵢ-BVᵢ, es kann jedoch auch BVᵢ-BNᵢ verwendet werden. Wichtig ist, daß innerhalb einer Auftragung eine einheitliche Differenzbildung verwendet wird.

Erfindungsgemäß wird eine Auftragung in der folgenden Weise vorgenommen:

In einem zweidimensionalen Koordinatensystem werden Punkte eingetragen, deren erste Koordinate Blutzuckerdifferenzen sind und deren zweite Koordinate die oben genannten Quotienten aus Insulineinheiten und Broteinheiten sind. In der Regel wird für die Darstellung ein kartesisches Koordinatensystem gewählt, dessen erste Achse beispielsweise von - 200 bis + 200 mg/dl reicht und dessen zweite Achse, die die erste Achse vorzugsweise am 0-Punkt schneidet. Die zweite Achse reicht beispielsweise von 0 bis 4.

Der Auftrag in einem derartigen Koordinatensystem liefert eine Punktwolke, deren Lage im Koordinatensystem und deren Streuung wichtige Aussagen für den Diabetiker bzw. den behandelnden Arzt liefert. Je dichter die Punkte der Punktwolke beisammen liegen, d.h. je geringer die Streuung ist, umso einheitlicher ist die Therapierung mit Insulin erfolgt. Dies bedeutet jedoch nicht, daß das Medikationsschema mit Insulin bereits optimal sein muß. Liegt nämlich diese Punktwolke weit vom Nullpunkt der ersten Koordinate (Differenzen der Blutglukosewerte) entfernt, so bedeutet dies, daß konsequent ein Therapieschema verfolgt wird, bei dem die Mahlzeit eine Verschiebung des Glukosespiegels verursacht.

Ziel der Insulinbehandlung ist es hingegen, den Einfluß der Mahlzeit auf den Blutzuckerspiegel möglichst gut zu kompensieren, d.h. einen Blutzuckerspiegel zu erzielen, der im Normbereich liegt. Im Rahmen der erfindungsgemäßen Auftragung von Punkten bedeutet dies, daß die ersten Koordinaten der Punkte möglichst nahe am Nullpunkt der ersten Achse liegen sollten.

Zur Vereinfachung der Auswertung kann der Schwerpunkt der Punktwolke errechnet und speziell gekennzeichnet in das Diagramm eingetragen werden. Die Koordinaten des Schwerpunktes ergeben sich aus dem Mittelwert der ersten Koordinate aller Punkte und dem Mittelwert der zweiten Koordinate aller Punkte. Bei einem gut eingestellten Diabetiker ist die erste Koordinate (Blutzuckerdifferenzen) des Schwerpunktes nahe Null und die Streuung der Punkte um den Schwerpunkt gering.

Mit Hilfe der erfindungsgemäßen Auftragung ist es weiterhin möglich, einen Vorschlag zur Verbesserung der Insulinmedikation zu gewinnen. Hierzu wird eine Regressionsgerade durch die Meßwerte berechnet und in das Diagramm eingezeichnet oder die Gleichung der Regressionsgerade numerisch wiedergegeben. Aus der Gleichung oder dem Diagramm wird der Wert der Reressionsgerade am Nullpunkt der ersten Koordinate ermittelt. Der so erhaltene Wert stellt einen Faktor dar, der im Folgenden als Sensitivitätsfaktor bezeichnet wird.

Die Ausgabe der erfindungsgemäßen Auftragung kann beispielsweise auf einem Display, einem Monitor oder auf einem Drucker erfolgen.

Das im Handel befindliche Insulin wird so gekennzeichnet, daß eine aufgenommene Broteinheit etwa durch eine Insulineinheit kompensiert werden kann (siehe hierzu Kap. 5.10 des o.g Buches "Diabetologie ..."). In der Praxis hängt jedoch die Zahl der Insulineinheiten, die zur Kompensation einer Broteinheit notwendig sind, von einer Reihe von Einflußfaktoren, wie z.B. der Art des Insulins, dem Körpergewicht der Person, der Empfindlichkeit der Person für Insulin, der Tageszeit und anderen Faktoren, ab.

Der Sensitivitätsfaktor ist ein Maß dafür, wieviele Insulineinheiten der jeweilige Diabetiker zur Kompensation einer bestimmten Zahl von Broteinheiten verwenden soll. Bisher wird der Sensitivitätsfaktor vom Diabetiker oder Arzt aufgrund der Erfahrungen für die jeweilige Person gewählt. Die Wahl des Sensitivitätsfaktors erfolgt bisher wenig systematisch und ist sehr zeitaufwendig, so daß die vorliegende Erfindung einen wesentlichen Fortschritt bringt, da die Wahl des Sensitivitätsfaktors systematisiert und verbessert werden kann.

Es ist mit Hilfe der erfindungsgemäßen Auftragung auch möglich, und wahrscheinlich für die größte der Zahl der Fälle auch besser, aus der Auftragung eine qualitative Korrektur der bisherigen Insulinmedikation abzuleiten. Hierzu wird zunächst der Schwerpunkt der Punktwolke ermittelt und aus seiner ersten Koordinate entnommen, ob er zu einem positiven oder negativen Differenzwert der Blutglukose gehört. Ist die erste Koordinate des Schwerpunktes positiv, so hat der Diabetiker im Mittel weniger Insulin gespritzt, als zur Erreichung des ursprünglichen Insulinwertes vor der Mahlzeit notwendig war. Hieraus ergibt sich die qualitative Anleitung, zukünftig etwas mehr Insulin pro Broteinheit zu verwenden als bisher. Für eine negative erste Koordinate des Schwerpunktes ergibt sich die inverse Anleitung.

Zur Sicherheit des Patienten sollte eine Veränderung der Insulintherapie jedoch nur vorgenommen werden, wenn die Streuung der Punkte in dem erfindungsgemäßen Diagramm ausreichend klein ist. Bei einer relativ großen Streuung ist hingegen davon auszugehen, daß schwer kalkulierbare Einflußfaktoren vorliegen, die eine Verläßlichkeit der Aussage stark herabsetzen. Eine Streuung der Punktwolke kann beispielsweise durch eine fehlerhafte oder ungenügend genaue Berechnung der mit der Mahlzeit aufgenommenen Broteinheiten verursacht werden. Dies gibt Veranlassung den Patienten zu schulen, so daß er konsequenter und genauer bei der Berechnung der aufgenommenen Broteinheiten vorgeht. Eine starke Streuung der Punktwolke kann jedoch auch durch Faktoren hervorgerufen werden, die außerhalb des Einflußbereiches des Patienten liegen. Beispielsweise kann der Insulinverbrauch pro Broteinheit physiologischen Schwankungen unterworfen sein. Die Möglichkeit anhand der Streuung die Notwendigkeit für Maßnahmen erkennen zu können, stellt einen großen Vorteil der Erfindung dar.

Die vorliegende Erfindung wird anhand der folgenden Beispiele näher erläutert:

Da sich die Insulinsensitivität mit der Tageszeit ändert, wurde der Tag in Zeitfenster eingeteilt, in denen die Sensitivität als weitestgehend gleichbleibend angenommen wird.

Die folgende Tabelle gibt ein Beispiel für diese Zeitfenster und ihre Bezeichnungen wieder:

| | |
|---|---|
| 4:30 Uhr bis 10:30 Uhr | Breakfast (BRK) - Lunch |
| 10:30 Uhr bis 15:00 Uhr | Lunch (LUN) - Dinner |
| 16:00 Uhr bis 21:00 Uhr | Dinner (DIN) - Bed |
| 21:00 Uhr bis 4:30 Uhr | Bed, Night (BED) - Breakfast |

Die folgende Tabelle gibt gemessene Blutglukosewerte wieder, die vor dem Frühstück (BRK) und vor Einnahme des Mittagessen (LUN) gemessen wurden. Die Tabelle enthält den Differenzwert (delta BG) und die darauffolgende Spalte den Quotienten aus Insulineinheiten und Broteinheiten für das zwischen den Messungen liegende Frühstück. In der vorletzten Spalte sind die zur Kompensation des Frühstücks verabreichten Insulineinheiten angegeben. Die letzte Spalte enthält die bei dem Frühstück eingenommene Anzahl von Broteinheiten.

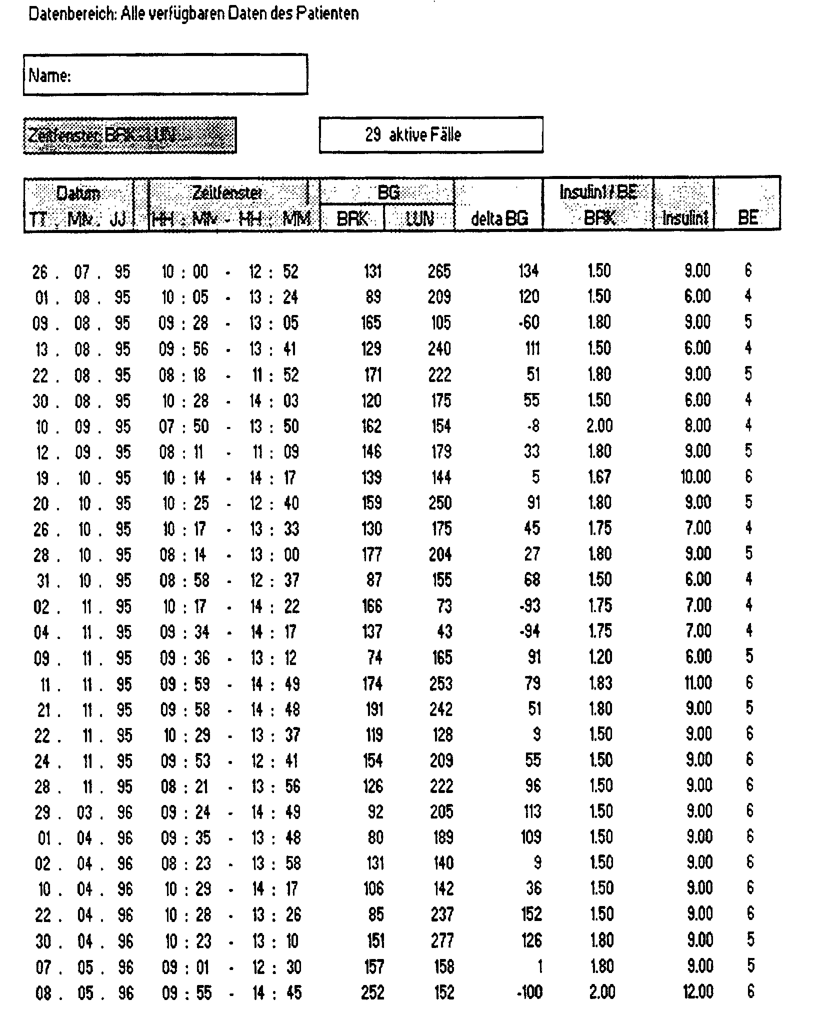

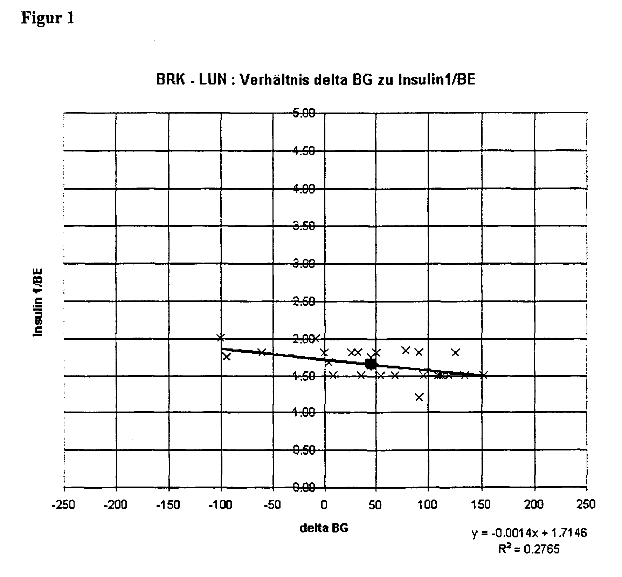

In der Figur 1 ist gemäß der folgenden Erfindung die Differenz der Blutglukosewerte (ΔBᵢ = BNᵢ-BVᵢ) gegen den Quotienten (Qᵢ=Iᵢ/BEᵢ) aus Insulindosis und Broteinheiten aufgetragen. Weiterhin ist in der Figur 1 der Schwerpunkt der Punktwolke sowie die Regressionsgerade zu erkennen. Aus der Figur ist ebenso zu erkennen, daß der Patient für eine Vielzahl von Medikationen einen Faktor im Bereich 1,2 bis 2,5 verwendet hat, um aus den Broteinheiten seine Insulindosis zu berechnen. Der relativ hohe ΔBᵢ-Bereich von - 100 bis + 180 zeigt an, daß der Patient relativ starr an bestimmten Insulindosen festhält, ohne die Menge der aufgenommenen Broteinheiten ausreichend bei der Wahl der Insulindosis zu berücksichtigen. Als Maßnahme zur besseren Einstellung dieses Diabetikers könnte daher eine Schulung durchgeführt werden, bei der er Informationen zur genaueren Berechnung der Broteinheiten und der zur Kompensation dieser Broteinheiten notwendigen Insulinmenge erhält.

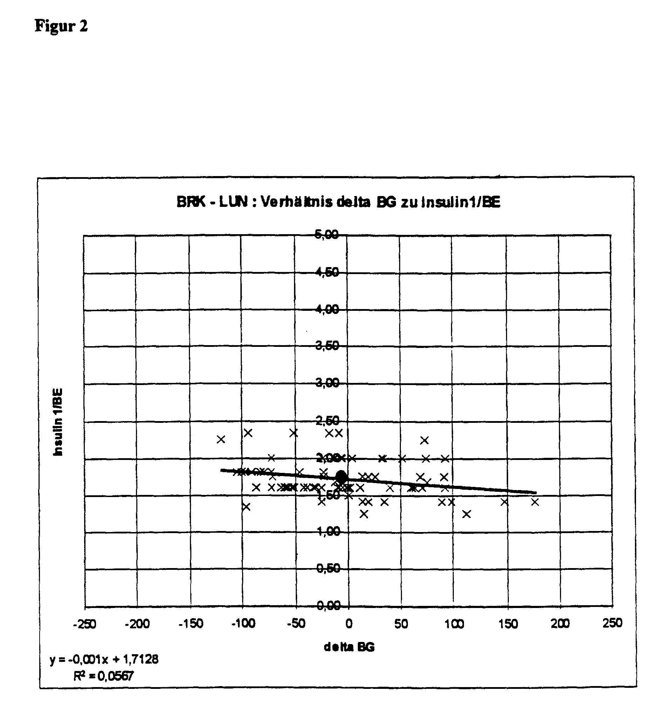

Figur 2 zeigt ein entsprechendes Diagramm, das die Kompensation des Frühstücks durch den gleichen Diabetiker wiedergibt. Es ist zu erkennen, daß der Schwerpunkt im Bereich des Nullpunktes liegt, was dafür spricht, daß der Diabetiker recht gut eingestellt ist.

Zur Erfindung gehört weiterhin eine Vorrichtung zur Durchführung der Kontrolle der Insulinmedikation. Eine derartige Vorrichtung beinhaltet eine Eingabeeinheit, mit der vom Bediener Blutzuckerwerte vor einer Mahlzeit, Blutzuckerwerte nach der Mahlzeit, bei der Mahlzeit aufgenommene Broteinheiten, zwischen den Messungen der Blutzuckerwerte verabreichte Insulineinheiten, sowie ggf. weitere Daten eingegeben werden können. Eine derartige Eingabeeinheit ist beispielsweise eine Tastatur. In einem weiter unten beschriebenen System kann die Eingabeeinheit auch eine Einheit zum Empfang von von einem Blutzuckermeßgerät ausgehenden Daten umfassen. Weiterhin besitzt die Vorrichtung eine Recheneinheit zur Berechnung von Blutzuckerdifferenzen aus den Blutzuckerwerten vor und nach einer Mahlzeit, sowie eine Recheneinheit zur Berechnung von Quotienten aus den Broteinheiten und den Insulineinheiten. Neben diesen Komponenten werden noch Speichereinheiten zur Speicherung der eingegebenen Daten und der berechneten Blutzuckerdifferenzen, sowie der Quotienten benötigt. Schließlich beinhaltet die Vorrichtung noch eine Ausgabeeinheit, wie ein Display, einen Bildschirm oder einen Drucker zur Ausgabe der weiter oben beschriebenen Koordinatensysteme mit Punkten, deren erste Koordinate Blutzuckerdifferenzen und deren zweite Koordinate die genannten Quotienten sind.

Weiterhin betrifft die vorliegende Erfindung noch ein System zur Kontrolle der Insulinmedikation, das die vorstehend genannte Vorrichtung, sowie ein Blutzuckermeßgerät umfaßt. Die Vorrichtung und das Blutzuckermeßgerät können getrennt vorliegen, so daß die gemessenen Blutzuckerwerte vom Benutzer manuell übertragen werden. Vorzugsweise sind Vorrichtung und Blutzuckermeßgerät jedoch über eine Datenleitung miteinander gekoppelt, so daß vom Blutzuckermeßgerät gemessene Werte direkt in die Vorrichtung übermittelt werden. Bei dieser Ausgestaltung ist es vorteilhaft, wenn der Benutzer eingeben kann, ob es sich bei der Messung um einen Blutzuckerwert vor oder nach der Mahlzeit handelt. Vorteilhafter Weise sind das Blutzuckermeßgerät und die Vorrichtung zusammen in einem Gehäuse untergebracht, so daß das System dem Benutzer als ein in seiner Funktion erweitertes Blutzuckermeßgerät erscheint.

## Patentansprüche

1. Verfahren zur Kontrolle der Insulinmedikation mit den Schritten
a) Zurverfügungstellen von Datensätzen mit den folgenden Daten:
- einen Blutzuckerwert (BVᵢ), der vor einer Mahlzeit ermittelt wurde,
- einen Blutzuckerwert (BNᵢ), der nach der Mahlzeit ermittelt wurde,
- die Broteinheiten (BEᵢ), die bei der Mahlzeit aufgenommen wurden,
- die Insulineinheiten (Iᵢ), die zwischen Ermittlung der Blutzuckerwerte BVᵢ und BNᵢ verabreicht wurden,
b) Bildung von Blutzuckerdifferenzen ΔBᵢ aus den Blutzuckerwerten vor und nach der Mahlzeit für die einzelnen Datensätze
c) Bildung von Quotienten Qᵢ aus Insulineinheiten (Iᵢ) und Broteinheiten (BEᵢ)
d) Auftragung von Punkten Pᵢ (ΔBᵢ; Qᵢ) in ein Koordinatensystem mit einer ersten Koordinate für die Blutzuckerdifferenzen (ΔBᵢ) und einer zweiten Koordinate für die Quotienten (Qᵢ)
e) Ausgabe des Koordinatensystems mit den Punkten auf einem Ausgabegerät.

2. Verfahren gemäß Anspruch 1, bei dem die Blutzuckerdifferenz als ΔBᵢ = BNᵢ-BVᵢ ermittelt wird.

3. Verfahren gemäß Anspruch 1, bei dem der Quotient (Qᵢ) als IᵢBEᵢ ermittelt wird.

4. Verfahren gemäß Anspruch 1, bei dem alle Blutzuckerwerte BVᵢ und BNᵢ vor bzw. nach dem jeweils gleichen Typ von Mahlzeit ermittelt wurden.

5. Verfahren gemäß Anspruch 2, bei dem der Typ von Mahlzeit aus der Gruppe Frühstück, Mittagessen und Abendessen ausgewählt ist.

6. Verfahren gemäß Anspruch 1, bei dem die Blutzuckerwerte BVᵢ maximal 1 Stunde vor Beginn der Mahlzeit und die Blutzuckerwerte BNᵢ spätestens vor der nächsten Mahlzeit bestimmt wurden.

7. Verfahren gemäß Anspruch 1, bei dem ein speziell gekennzeichneter Schwerpunkt mit dem Koordinatensystem ausgegeben wird.

8. Verfahren gemäß Anspruch 1 oder 7, bei dem eine Regressionsgerade aus den Punkten ermittelt wird.

9. Verfahren gemäß Anspruch 8, bei dem die Regressionsgerade zusammen mit den Punkten und dem Koordinatensystem ausgegeben wird.

10. Verfahren gemäß Anspruch 8 oder 9, bei dem der Wert der Regressionsgeraden für ΔBᵢ = 0, , entweder graphisch oder numerisch ausgegeben wird.

11. Verfahren gemäß Anspruch 7, bei dem die erste Koordinate des Schwerpunktes qualitativ oder quantitativ ausgegeben wird.

12. Verfahren gemäß Anspruch 1, bei dem eine Überprüfung erfolgt, ob der Quotient (Qᵢ) positiv ist und nur solche Datensätze verwendet werden, für die dies zutrifft.

13. Verfahren gemäß Anspruch 1, bei dem eine Überprüfung erfolgt, ob der Quotient (Qᵢ) im Bereich von 0.1 bis 10 liegt und nur solche Datensätze verwendet werden, für die dies zutrifft.

14. Verfahren gemäß Anspruch 1, bei dem eine Überprüfung erfolgt, ob der Blutzuckerwert vor der Mahlzeit (BVᵢ) im Bereich von 80 bis 180 mg/dl liegt und nur solche Datensätze verwendet werden, für die dies zutrifft.

15. Verfahren gemäß Anspruch 1, bei dem eine Überprüfung erfolgt, ob der Quotient (Qᵢ) im Bereich von 0. 1 bis 10 liegt und nur solche Datensätze verwendet werden, für die dies zutrifft.

16. Vorrichtung zur Kontrolle der Insulinmedikation mit folgenden Einheiten:
eine Eingabeeinheit zur Eingabe von
- Blutzuckerwerten (BVᵢ), die vor einer Mahlzeit ermittelt wurden,
- Blutzuckerwerten (BNᵢ), der nach der Mahlzeit ermittelt wurden,
- Broteinheiten (BEᵢ), die bei der Mahlzeit aufgenommen wurden,
- Insulineinheiten (Iᵢ), die zwischen Ermittlung der Blutzuckerwerte BVᵢ und BNᵢ verabreicht wurden,
eine Einheit zur Bildung von Blutzuckerdifferenzen ΔBᵢ aus den Blutzuckerwerten vor und nach der Mahlzeit für die einzelnen Datensätze,
eine Einheit zur Bildung von Quotienten Qᵢ aus Insulineinheiten (Iᵢ) und Broteinheiten (BEᵢ)
eine Ausgabeeinheit zur Ausgabe einer Auftragung von Punkten Pᵢ (ΔBᵢ; Qᵢ) in ein Koordinatensystem mit einer ersten Koordinate für die Blutzuckerdifferenzen (ΔBᵢ) und einer zweiten Koordinate für die Quotienten (Qᵢ).

17. System zur Kontrolle der Insulinmedikation beinhaltet eine Vorrichtung gemäß Anspruch 16, sowie ein Blutzuckermeßgerät.

18. System gemäß Anspruch 17, bei dem die Vorrichtung und das Blutzuckermeßgerät über eine Datenleitung miteinander verbunden sind, so daß das Blutzuckermeßgerät Blutzuckerwerte an die Vorrichtung übertragen kann.

19. System gemäß Anspruch 18, bei dem die Vorrichtung und das Blutzuckermeßgerät gemeinsam in einem Gehäuse untergebracht sind.

## Claims

1. Method for monitoring insulin medication comprising the steps
a) providing sets of data containing the following data:
- a blood sugar value (BVᵢ), which was determined before a meal,
- a blood sugar value (BNᵢ), which was determined after a meal,
- the bread units (BEᵢ) which were ingested during the meal,
- the insulin units (Iᵢ) which were administered between the determination of the blood sugar values BVᵢ and BNᵢ,
b) calculating blood sugar differences ΔBᵢ from the blood sugar values before and after the meal for the individual sets of data,
c) calculating a quotient Qᵢ from the insulin units (Iᵢ) and bread units BEᵢ,
d) plotting points Pᵢ (ΔBᵢ; Qᵢ) in a system of coordinates comprising a first coordinate for the blood sugar differences (ΔBᵢ) and a second coordinate for the quotients (Qᵢ),
e) output of the coordinate system with the points on an output device.

2. Method as claimed in claim 1, in which the blood sugar difference is determined as ΔBᵢ=BNᵢ-BVᵢ.

3. Method as claimed in claim 1, in which the quotient (Qᵢ) is determined as Iᵢ/BEᵢ.

4. Method as claimed in claim 1, in which all blood sugar values BVᵢ and BNᵢ have been determined before or after the same type of meal in each case.

5. Method as claimed in claim 2, in which the type of meal is selected from the group breakfast, lunch and dinner.

6. Method as claimed in claim 1, in which the blood sugar values BVᵢ have been determined no more than 1 hour before the start of the meal and the blood sugar values BNᵢ have been determined at the latest before the next meal.

7. Method as claimed in claim 1, in which a specially labelled centre of concentration is displayed with the system of coordinates.

8. Method as claimed in claim 1 or 7, in which a regression line is determined from the points.

9. Method as claimed in claim 8 or 9, in which the regression line is displayed together with the points and the system of coordinates.

10. Method as claimed in claim 8 or 9, in which the value of the regression line for ΔBᵢ=0 is either displayed graphically or numerically.

11. Method as claimed in claim 7, in which the first coordinates of the centre of concentration is shown qualitatively or quantitatively.

12. Method as claimed in claim 1, in which it is checked whether the quotient (Qᵢ) is positive and only those sets of data are used for which this applies.

13. Method as claimed in claim 1, in which it is checked whether the quotient (Qᵢ) is in the range of 0.1 to 10 and only those sets of data are used for which this applies.

14. Method as claimed in claim 1, in which it is checked whether the blood sugar value before the meal (BVᵢ) is in the range of 80 to 180 mg/dl and only those sets of data are used for which this applies.

15. Method as claimed in claim 1, in which it is checked whether the quotient (Qᵢ) is in the range of 0.1 to 10 and only those sets of data are used for which this applies.

16. Device for monitoring insulin medication comprising the following units:
an input unit to input
- blood sugar values (BVᵢ), which were determined before a meal,
- blood sugar values (BNᵢ), which were determined after a meal,
- bread units (BEᵢ) which were ingested during the meal,
- insulin units (Ii) which were administered between the determination the blood sugar values BVᵢ and BNᵢ,
a unit for calculating blood sugar differences ΔBᵢ from the blood sugar values before and.after the meal for the individual sets of data,
a unit for calculating quotients Qᵢ from insulin units (Iᵢ) and bread units BEᵢ,
an output unit to display a graph of points Pᵢ (ΔBᵢ; Qᵢ) in a system of coordinates in which the first coordinates are the blood sugar differences (ΔBᵢ) and the second coordinates are the quotients (Qᵢ).

17. System for monitoring insulin medication comprising a device as claimed in claim 16 and a blood sugar measuring instrument.

18. System as claimed in claim 17, in which the device and the blood sugar measuring instrument are coupled together by a dataline such that the blood sugar measuring instrument can transmit blood sugar values to the device.

19. System as claimed in claim 18, in which the device and the blood sugar measuring instrument are accommodated in a common housing.

## Revendications

1. Procédé pour contrôler la médication à l'insuline avec les étapes :
a) mise à disposition d'enregistrements de données avec les données suivantes :
- une valeur de glycémie (BVᵢ), qui a été déterminée avant un repas,
- une valeur de glycémie (BNᵢ), qui a été déterminée après le repas,
- les unités glucidiques (BEᵢ), qui ont été absorbées pendant le repas,
- les unités d'insuline (Lᵢ), qui ont été administrées entre la détermination des valeurs de glycémie BVᵢ et BNᵢ,
b) formation des différences de glycémie ΔBᵢ à partir des valeurs de glycémie avant et après le repas pour les enregistrements de données individuels
c) formation de quotients Qᵢ à partir des unités d'insuline (Iᵢ) et des unités glucidiques (BEᵢ)
d) report des points Pᵢ (ΔBᵢ; Qᵢ) dans un système de coordonnées ayant une première coordonnée pour les différences de glycémie (ΔBᵢ) et une deuxième coordonnée pour les quotients (Qᵢ)
e) émission du système de coordonnées avec les points sur une unité de sortie.

2. Procédé selon la revendication 1, dans lequel la différence de glycémie est déterminée sous la forme ΔBᵢ = BNᵢ - BVᵢ.

3. Procédé selon la revendication 1, dans lequel le quotient (Qᵢ) est déterminé sous la forme de Iᵢ/BEᵢ.

4. Procédé selon la revendication 1, dans lequel toutes les valeurs de glycémie BVᵢ et BNᵢ, ont été déterminées avant ou bien après le même type respectif de repas.

5. Procédé selon la revendication 2, dans lequel le type de repas est choisi dans le groupe formé par le petit déjeuner, le déjeuner et le repas du soir.

6. Procédé selon la revendication 1, dans lequel les valeurs de glycémie BVᵢ ont été déterminées au maximum une heure avant le début du repas et les valeurs de glycémie BNᵢ, au plus tard avant le repas suivant.

7. Procédé selon la revendication 1, dans lequel un barycentre spécialement marqué avec le système de coordonnées est émis.

8. Procédé selon la revendication 1 ou 7, dans lequel une droite de régression est déterminée à partir des points.

9. Procédé selon la revendication 8, dans lequel la droite de régression conjointement avec les points et le système de coordonnées est émise.

10. Procédé selon la revendication 8 ou 9, dans lequel la valeur des droites de régression pour ΔBᵢ = 0, est émise graphiquement ou numériquement.

11. Procédé selon la revendication 7, dans lequel la première coordonnée du barycentre est émise de façon qualitative ou quantitative.

12. Procédé selon la revendication 1, dans lequel se réalise un contrôle pour savoir si le quotient (Qᵢ) est positif, et ne sont employés que les enregistrements de données pour lesquels ceci est vrai.

13. Procédé selon la revendication 1, dans lequel se réalise un contrôle pour savoir si le quotient (Qᵢ) se trouve dans la plage de 0,1 à 10, et ne sont employés que les enregistrements de données pour lesquels ceci est vrai.

14. Procédé selon la revendication 1, dans lequel se réalise un contrôle pour savoir si la valeur de glycémie avant le repas (BVᵢ) se trouve dans la plage de 80 à 180 mg/dl, et ne sont employés que les enregistrements de données pour lesquels ceci est vrai.

15. Procédé selon la revendication 1, dans lequel se réalise un contrôle pour savoir, si le quotient (Qᵢ) se trouve dans la plage de 0,1 à 10, et ne sont employés que les enregistrements de données pour lesquels ceci est vrai.

16. Dispositif pour contrôler la médication à l'insuline, ayant les unités suivantes
une unité de saisie pour saisir
- les valeurs de glycémie (BVᵢ), qui ont été déterminées avant un repas,
- les valeurs de glycémie (BNᵢ), qui ont été déterminées après le repas,
- les unités glucidiques (BEᵢ), qui ont été absorbées pendant le repas,
- les unités d'insuline (Iᵢ), qui ont été administrées entre la détermination des valeurs de glycémie BVᵢ et BNᵢ,
une unité pour former des différences de glycémie ΔBi à partir des valeurs de glycémie avant et après le repas pour les enregistrements de données individuels,
une unité pour former des quotients Qᵢ à partir des unités d'insuline (Ii) et des unités glucidiques (BEᵢ)
une unité de sortie pour émettre un relevé des points Pᵢ (ΔBᵢ; Qᵢ) dans un système de coordonnées avec une première coordonnée pour les différences de glycémie (ΔBᵢ) et une deuxième coordonnée pour les quotients (Qᵢ).

17. Système pour contrôler la médication à l'insuline contenant un dispositif selon la revendication 16, ainsi qu'un appareil de mesure de glycémie.

18. Système selon la revendication 17, dans lequel le dispositif et l'appareil de mesure de glycémie sont reliés entre eux par une ligne de transmission de données, de sorte que l'appareil de mesure de glycémie puisse transmettre les valeurs de glycémie au dispositif.

19. Système selon la revendication 18, dans lequel le dispositif et l'appareil de mesure de glycémie sont logés ensemble dans un boîtier.
